Europäisches Patentamt

European Patent Office

Office européen des brevets

(19)

(11) Veröffentlichungsnummer: **0 036 132**

A1

(12) **EUROPÄISCHE PATENTANMELDUNG**

(21) Anmeldenummer: **81101542.9**

(22) Anmeldetag: **04.03.81**

(51) Int. Cl.³: **A 61 B 5/02**
**A 61 M 25/00**

(30) Priorität: **13.03.80 DE 8006872 U**

(43) Veröffentlichungstag der Anmeldung:
**23.09.81 Patentblatt 81/38**

(84) Benannte Vertragsstaaten:
**BE CH FR GB IT LI LU NL**

(71) Anmelder: **Nessler, Reiner, Dr.**
**Paracelsiusstrasse 24**
**D-3320 Salzgitter-Bad(DE)**

(72) Erfinder: **Nessler, Reiner, Dr.**
**Paracelsiusstrasse 24**
**D-3320 Salzgitter-Bad(DE)**

(74) Vertreter: **Gramm, Werner, Dipl.-Ing. et al,**
**Patentanwälte Gramm + Lins Theodor-Heuss-Strasse 2**
**D-3300 Braunschweig(DE)**

(54) **Venenkatheter.**

(57) Die Erfindung betrifft ein Venenkatheter zur Messung des zentralen Venendrucks in der oberen Hohlvene, bestehend aus einem in die Vene einzuführenden flexiblen Kunststoffschlauch (1), dessen eines Ende in ein Ansatzstück (2) größeren Durchmessers mit einer Griffplatte (4) zur Handhabung und Festlegung des Katheters übergeht, wobei die Griffplatte (4) oberhalb der Verbindungsstelle zwischen Kunststoffschlauch (1) und Ansatzstück (2) an letzterem sitzt und in einer zumindest angenähert senkrecht zu der Schlauchachse verlaufenden Ebene liegt.

Als neu wird angesehen, daß der Kunststoffschlauch (1) durch eine Fixationsplatte (5) geführt ist, die streifenförmig ausgebildet ist, in einer Ebene senkrecht zur Ebene der Griffplatte (4) liegt und den Kunststoffschlauch beidseitig überragt.

EP 0 036 132 A1

Dr. Reiner Nessler
Paracelsiusstr. 24
3320 Salzgitter-Bad

Telefon:      (05 31) 8 00 79
Telex:        09 52 620

Anwaltsakte   231-2 EP-1
Datum         3.3.1981

"Venenkatheter"

Die Neuerung betrifft ein Venenkatheter zur Messung des zentralen Venendrucks in der oberen Hohlvene, bestehen aus einem in die Vene einzuführenden flexiblen Kunststoffschlauch, dessen eines Ende in ein Ansatzstück größeren Durchmessers mit einer Griffplatte zur Handhabung und Festlegung des Katheters übergeht, wobei die Griffplatte oberhalb der Verbindungsstelle zwischen Kunststoffschlauch und Ansatzstück an letzterem sitzt und in einer zumindest angenähert senkrecht zu der Schlauchachse verlaufenden Ebene liegt.

Diese Ausführungsform ist durch das DE-GM 75 30 163 bekanntgeworden. Hier dient die Griffplatte zugleich als Anschlag für den eingeführten Katheter. Hierdurch ist die exakte Lage des Venenkatheters ohne Schwierigkeit reproduzierbar. Dies führt zu dem großen Vorteil, daß für eine jeweils gleiche Einführungsstelle für den Katheter dessen Kunststoffschlauch immer die gleiche Länge aufweist. Der Schlauch braucht daher nicht mehr durch Abschneiden der überschüssigen Länge individuell angepaßt zu werden.

- 2 -

Diese Ausführungsform hat sich in der Praxis hervorragend bewährt. Der Neuerung liegt die Aufgabe zugrunde, den eingangs
erläuterten Venenkatheter hinsichtlich seiner Befestigungsmöglichkeit noch weiter zu verbessern.

Diese Aufgabe wird gemäß der Neuerung dadurch gelöst, daß der
Kunststoffschlauch durch eine Fixationsplatte geführt ist, die
streifenförmig ausgebildet ist, in einer Ebene senkrecht zur
Ebene der Griffplatte liegt und den Kunststoffschlauch beidseitig überragt.

Durch einfaches Überkleben der beiden den Kunststoffschlauch
überragenden Enden der Fixationsplatte mit einem Pflaster läßt
sich der Venenkatheter schnell, einfach und sicher befestigen.
Irgendwelche aus Polster, Sicherheitsnadel u.dgl. bestehende
Hilfskonstruktionen sind überflüssig. Die Fixationsplatte verhindert überdies ein Eindrücken der Verbindungsstelle zwischen
Kunststoffschlauch und Ansatzstück in die Haut des Patienten.

Dabei ist es vorteilhaft, wenn die Fixationsplatte die Verbindungsstelle zwischen Kunststoffschlauch und Ansatzstück etwas
übergreift. Dadurch erhält man einen verbesserten Schutz gegen
ein mögliches Abknicken des Kunststoffschlauchs an der Verbindungsstelle.

Die Fixationsplatte besteht zweckmäßig aus elastischem Material,
vorzugsweise Schaumgummi, das sich der Haut des Patienten anschmiegt, und von dem sich das Befestigungspflaster leicht lösen
läßt.

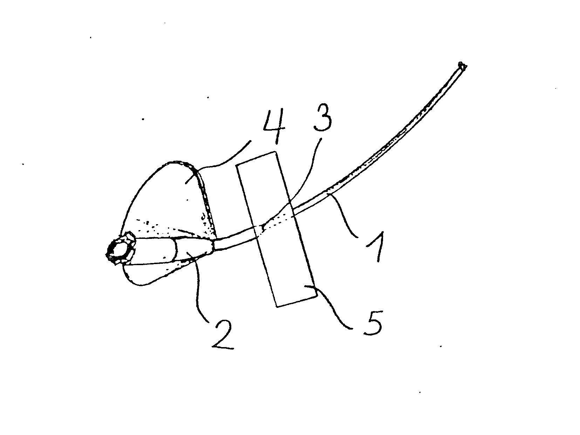

In der Zeichnung ist eine beispielsweise Ausführungsform der
Neuerung perspektivisch dargestellt.

Danach besteht der dargestellte Venenkatheter aus einem in die
Vene einzuführenden flexiblen Kunststoffschlauch 1, dessen eines
Ende in ein Ansatzstück 2 größeren Durchmessers übergeht. Oberhalb der Verbindungsstelle 3 zwischen Kunststoffschlauch 1 und
Ansatzstück 2 sitzt an letzterem eine Griffplatte 4, die etwa
flügelförmig ausgebildet ist und in einer senkrecht zu der
Schlauchachse verlaufenden Ebene liegt.

Auf den Kunststoffschlauch 1 ist eine Fixationsplatte 5 aufgeschoben, die streifenförmig ausgebildet ist, in einer Ebene
senkrecht zur Ebene der Griffplatte 4 liegt und den Kunststoffschlauch beidseitig überragt. Dabei ist die Fixationsplatte so
weit auf den Kunststoffschlauch 1 aufgeschoben, daß sie die Verbindungsstelle 3 zum Ansatzstück 2 hin etwas übergreift.

Gr/Gru.

Dr. Reiner Nessler

Paracelsiusstr. 24

3320 Salzgitter-Bad

Telefon: (05 31) 8 00 79
Telex: 09 52 620

Anwaltsakte 231-2 EP-1
Datum 3.3.1931

Patentansprüche:

1. Venenkatheter zur Messung des zentralen Venendrucks in der oberen Hohlvene, bestehend aus einem in die Vene einzuführenden flexiblen Kunststoffschlauch, dessen eines Ende in ein Ansatzstück größeren Durchmessers mit einer Griffplatte zur Handhabung und Festlegung des Katheters übergeht, wobei die Griffplatte oberhalb der Verbindungsstelle zwischen Kunststoffschlauch und Ansatzstück an letzterem sitzt und in einer zumindest angenähert senkrecht zu der Schlauchachse verlaufenden Ebene liegt, d a d u r c h  g e k e n n z e i c h n e t, daß der Kunststoffschlauch (1) durch eine Fixationsplatte (5) geführt ist, die streifenförmig ausgebildet ist, in einer Ebene senkrecht zur Ebene der Griffplatte (4) liegt und den Kunststoffschlauch beidseitig überragt.

2. Venenkatheter nach Anspruch 1, dadurch gekennzeichnet, daß die Fixationsplatte (5) die Verbindungsstelle (3) zwischen Kunststoffschlauch (1) und Ansatzstück (2) etwas übergreift.

3. Venenkatheter nach Anspruch 1 oder 2, dadurch gekennzeichnet, daß die Fixationsplatte (5) aus elastischem Material, vorzugsweise Schaumgummi besteht.

Patentanwälte

G r a m m  +  L i n s

Gr/Gru.

0036132

| | Europäisches Patentamt | EUROPÄISCHER RECHERCHENBERICHT | Nummer der Anmeldung<br>EP 81 10 1542 |

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | A 61 B 5/02<br>A 61 M 25/00 |
| D | <u>DE - U - 75 30 163</u> (R. NESSLER)<br>* ganzes Dokument * | 1 | |
| | -- | | |
| | <u>US - A - 4 161 177</u> (H. FUCHS)<br>* Zusammenfassung; Spalte 3, Zeile 27 bis Spalte 4, Zeile 14; Figuren 1,2 *<br>& AT - A - 356 798 | 1,2 | |
| | -- | | **RECHERCHIERTE SACHGEBIETE (Int. Cl.³)** |
| | <u>US - A - 3 834 380</u> (W.A. BOYD)<br>* Zusammenfassung; Spalte 1, Zeilen 30-38; Spalte 2, Zeile 41 bis Spalte 3, Zeile 36; Figuren 1-5 * | 1,2 | A 61 B 5/02<br>A 61 M 25/00<br>25/02 |
| | -- | | |
| | <u>GB - A - 1 243 576</u> (ABBOTT LABORATORIES)<br>* Seite 2, Zeilen 27-41; Seite 2, Zeile 91 bis Seite 3, Zeile 17; Seite 3, Zeile 124 - Seite 4, Zeile 54; Figuren 1,2,5-11 *<br>& DE - A - 1 929 050 | 1,2 | |
| | -- | | **KATEGORIE DER GENANNTEN DOKUMENTE** |
| A | <u>US - A - 1 642 819</u> (J.E. LONG)<br>* Seite 1, Zeilen 1-14; Seite 1, Zeilen 39-83; Figuren 1-4 * | 1 | X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| | -- | | |
| | ./.. | | |
| | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Den Haag | 19.06.1981 | BEAVEN |

EPA form 1503.1 06.78

Europäisches
Patentamt

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| P | EP - A - 0 023 580 (INTERMEDICAT GmbH) <br><br> * Zusammenfassung; Seite 9, Zeile 21 bis Seite 11, Zeile 12; Seite 13, Zeile 1 bis Seite 14, Zeile 22; Figuren 1-5,20-24 * | 1,2 |

------

**KLASSIFIKATION DER ANMELDUNG (Int.Cl. 3)**

**RECHERCHIERTE SACHGEBIETE (Int. Cl. 3)**